(19) Europäisches Patentamt
European Patent Office
Office européen des brevets

(11) Veröffentlichungsnummer: **0 570 831 A2**

(12) # EUROPÄISCHE PATENTANMELDUNG

(21) Anmeldenummer: **93107735.8**

(51) Int. Cl.[5]: **C07D 473/06**, A61K 31/52

(22) Anmeldetag: **12.05.93**

(30) Priorität: **20.05.92 DE 4216711**

(43) Veröffentlichungstag der Anmeldung:
**24.11.93 Patentblatt 93/47**

(84) Benannte Vertragsstaaten:
**AT BE CH DE DK ES FR GB GR IE IT LI LU MC NL PT SE**

(71) Anmelder: **HOECHST AKTIENGESELLSCHAFT**

**D-65926 Frankfurt(DE)**

(72) Erfinder: **Thorwart, Werner, Dr.**
**Am Gänseborn 3**
**W-6203 Hochheim/Main(DE)**
Erfinder: **Furrer, Harald, Dr.**
**Wilhelm-Leuschner-Strasse 7**
**W-6238 Hofheim/Ts.(DE)**
Erfinder: **Wolf, Erhard, Dr.**
**Ostpreussenstrasse 17**
**W-6238 Hofheim (Ts)(DE)**
Erfinder: **Gebert, Ulrich, Dr.**
**Am Höhenstrauch 14**
**W-6246 Glashütten/Ts.(DE)**
Erfinder: **Rossmanith, Erhard**
**Spechtstrasse 4**
**65824 Schwalbach(DE)**
Erfinder: **Grome, John J., Dr.**
**Fritz-Reuter-Strasse 1**
**W-6200 Wiesbaden(DE)**
Erfinder: **Schneider, Ernst-Jürgen, Dr.**
**Heinrich-Held-Strasse 22**
**W-6277 Bad Camberg 2(DE)**

(54) **Verwendung von Xanthinderivaten zur Behandlung von Nervenschädigungen nach Unterbrechung der Blutzirkulation.**

(57) Die Erfindung betrifft die Verwendung von Xanthinderivaten der Formel I

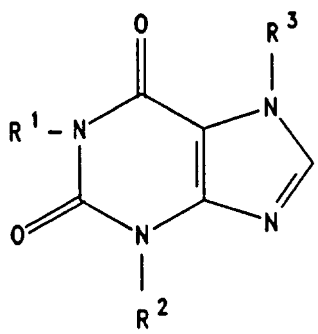

(I)

wobei $R^2$ eine ($C_1$-$C_4$)-Alkyl ist und mindestens eines der Symbole $R^1$ und $R^3$ für den Rest der Formel II oder III

EP 0 570 831 A2

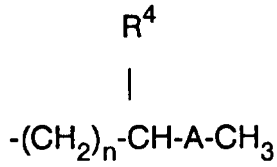

(II)

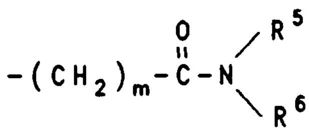

(III)

steht, wobei A CHOH, CO oder Dioxolan, $R^4$ Wasserstoffatom oder ($C_1$-$C_4$)-Alkyl, n 0 bis 5 ist wobei $R^5$ und $R^6$ Wasserstoffatom oder ($C_1$-$C_4$)-Alkyl bedeuten oder zusammen mit dem Stickstoffatom an das sie gebunden sind, einen 5 bis 7-gliedrigen Ring bilden, wobei ein C-Atom durch ein Sauerstoff- oder Stickstoffatom ersetzt sein kann, m 1 oder 2 ist und der gegebenenfalls andere Rest $R^1$ oder $R^3$, Wasserstoffatom,
($C_1$-$C_6$)-Alkyl oder ($C_3$-$C_6$)-Alkenyl bedeutet, zur Herstellung von Arzneimitteln zur Prophylaxe und Behandlung von Nervenschädigungen nach Unterbrechung der Blutzirkulation und neue Xanthinderivate, sowie Verfahren zu ihrer Herstellung.

Eine Reihe von Oxoalkyl- und Hydroxyalkylxanthinen wirkt durchblutungsfördernd und kann auch bei cerebralen Durchblutungsstörungen eingesetzt werden (US 4,289,776, PCT 86/00401, US 3,737,433). Es sind auch Verfahren zur Herstellung der obengenannten Xanthinderivate bekannt (US 4,289,776; US 3,737,433).

Bisher sind keine Arzneimittel bekannt, die es erlauben, die Zeit für eine symptomfreie Unterbrechung der Blutzirkulation zu verlängern oder die zu einer signifikanten Verminderung, der durch die Unterbrechung der Blutzirkulation bedingten Nervenschädigungen führen. Eine erste Folge aufgrund der Unterbrechung der Blutzirkulation ist das zu Stoffwechselstörungen führende Sauerstoffmangelangebot in den davon betroffenen Arealen.

Es wurde nun gefunden, daß die erfindungsgemäßen Xanthinderivate geeignet sind, die Zeit für eine symptomfreie Unterbrechung der Blutzirkulation zu verlängern, die Nervenschädigungen die nach Unterbrechung der Blutzirkulation auftreten mindern und den Sauerstoffpartialdruck im Gehirn steigern.

Die Erfindung betrifft daher die Verwendung von mindestens einem Xanthinderivat der Formel I

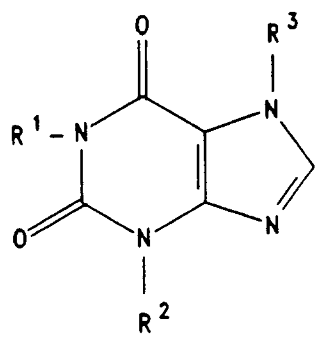

(I)

wobei $R^2$ eine Alkylgruppe mit 1 bis 4 C-Atomen ist und

a) mindestens einer der Reste $R^1$ und $R^3$ einen geradkettigen oder verzweigten Rest der Formel II

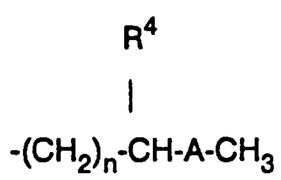

(II)

darstellt, worin A

1) 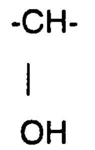 ,

2) 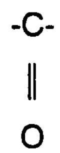 oder

3) 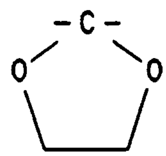

bedeutet,

$R^4$

1) Wasserstoffatom oder

2) $(C_1-C_4)$-Alkylgruppe ist und

n eine ganze Zahl von 0 bis 5 darstellt oder

b) mindestens einer der Reste $R^1$ und $R^3$ ein Rest der Formel III

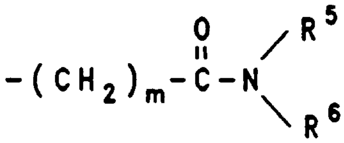 (III)

darstellt, worin

$R^5$ und $R^6$ gleich oder verschieden sind und unabhängig voneinander ein Wasserstoffatom oder eine Alkylgruppe mit 1 bis 4 C-Atomen, oder $R^5$ und $R^6$ zusammen mit dem Stickstoffatom an das sie gebunden sind, einen 5 bis 7-gliedrigen Ring bilden, der gegebenenfalls anstelle eines der ringständigen C-Atome ein weiteres Heteroatom in Form von Sauerstoff oder Stickstoff enthält, wobei das weitere Stickstoffatom auch durch eine $(C_1-C_4)$-Alkylgruppe substituiert sein kann,

m die ganze Zahl 1 oder 2 ist

und der gegebenenfalls vorhandene andere Rest $R^1$ oder $R^3$ steht für

1) Wasserstoffatom,

2) $(C_1-C_6)$-Alkyl, geradkettig oder verzweigt,

3) $(C_1-C_6)$-Alkyl, geradkettig oder verzweigt, worin die Kohlenstoffkette mit 1 oder 2 nicht aneinander gebundenen Sauerstoffatomen unterbrochen ist oder

4) $(C_3-C_6)$-Alkenyl, geradkettig oder verzweigt,

wobei 1-(5-Oxohexyl)-3-methyl-7-n-propylxanthin ausgenommen ist, zur Herstellung von Arzneimitteln zur Prophylaxe und Behandlung von Nervenschädigungen nach Unterbrechung der Blutzirkulation.

Die Unterbrechung der Blutzirkulation kann durch Abschnürbinden oder Abschnürklemmen erfolgen um beispielsweise ein blutfreies Operationsfeld zu erhalten sowie durch arterielle oder venöse Okklusion oder Verschluß aufgrund embolischer oder thrombotischer Verschlüße in Blutgefäßen. Zur Unterbrechung der Blutzirkulation kommt es beispielsweise bei gefäßchirurgischen Operationen wie Entfernung von Tromben, bei Transplantationen von Organen wie Herz oder Niere sowie bei Verschlüßen in Arterien oder Venen wie sie beispielsweise nach Schlaganfall, Gehirnschlag oder Herzinfarkt auftreten. Je nach Dauer der Blutzirkulationsunterbrechung kommt es zu mehr oder weniger starken Nervenschädigungen, die von leichten Nervenleistungsstörungen: bis zum völligen Ausfall der Nerven, wie z. B. Lähmungen, reichen können. Als

Symptome dieser Nervenschädigungen seien beispielsweise genannt: Membranpotentialstörungen der Nerven, geistige Verwirrung der Patienten, Orientierungslosigkeit, fehlende Ansprechbarkeit des Patienten, Gedächtnisstörungen, mangelnde Konzentrationsfähigkeit, motorische Störungen oder Lähmungen. Die Blutzirkulation kann völlig oder teilweise unterbrochen sein und dadurch bedingt werden mehr oder weniger starke Nervenschädigungen zu beobachten sein.

Bevorzugt werden Xanthinderivate der Formel I wobei $R^2$ eine Alkylgruppe mit 1 bis 2 C-Atomen ist und mindestens einer der Reste $R^1$ und $R^3$ einen geradkettigen oder verzweigten Rest der Formel II darstellt, worin A

1) 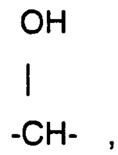 ,

2) 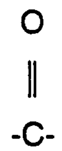 oder

3) 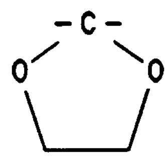

bedeutet,

$R^4$ Wasserstoffatom oder eine ($C_1$-$C_2$)-Alkylgruppe ist und

n eine ganze Zahl von 2 bis 5 darstellt

und der gegebenenfalls vorhandene andere Rest $R^1$ oder $R^3$ steht für

1) Wasserstoffatom,

2) ($C_1$-$C_6$)-Alkyl, geradkettig oder verzweigt,

3) ($C_1$-$C_6$)-Alkyl, geradkettig oder verzweigt, worin die Kohlenstoffkette mit 1 oder 2 Sauerstoffatomen unterbrochen ist,

4) ($C_3$-$C_6$)-Alkenyl, geradkettig oder verzweigt,

wobei 1-(5-Oxohexyl)-3-methyl-7-n-propylxanthin ausgenommen ist, eingesetzt.

5- bis 7-gliedrige Ringe sind z.B. Pyrrol, Pyrrolin, Pyrrolidin, Pyridin, Tetrahydropyridin, Piperidin, Azepin, Pyrazol, Imidazol, Pyrazolin, Imidazolin, Pyrazolidin, Imidazolidin, Pyridazin, Pyrimidin, Pyrazin, Piperazin, Diazepin, Oxazol, Isoxazol, 2-Isoxazolin, Isoxazolidin oder Morpholin.

Die Erfindung betrifft ferner neue Xanthinderivate der Formel I, wobei $R^2$ eine Alkylgruppe mit 1 bis 4 C-Atomen ist und

a) mindestens einer der Reste $R^1$ und $R^3$ einen geradkettigen oder verzweigten Rest der Formel II darstellt, worin A

1) 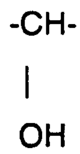 ,

2) 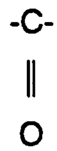 oder

3) 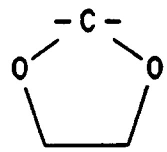

bedeutet,

$R^4$ eine $(C_1-C_4)$-Alkylgruppe ist und

n eine ganze Zahl von 2 bis 5 darstellt oder

b) mindestens einer der Reste $R^1$ und $R^3$ einen Rest der Formel III

darstellt, worin

$R^5$ und $R^6$ gleich oder verschieden sind und unabhängig voneinander ein Wasserstoffatom oder eine $(C_1-C_4)$-Alkylgruppe sind, oder $R^5$ und $R^6$ zusammen mit dem Stickstoffatom an das sie gebunden sind, einen 5 bis 7-gliedrigen Ring bilden, der gegebenenfalls anstelle eines der ringständigen C-Atome ein weiteres Heteroatom in Form von Sauerstoff oder Stickstoff enthält, wobei das weitere Stickstoffatom auch durch eine $(C_1-C_4)$-Alkylgruppe substituiert sein kann,

m die ganze Zahl 1 oder 2 ist

und der gegebenenfalls vorhandene andere Rest $R^1$ oder $R^3$ steht für

1) Wasserstoffatom,

2) $(C_1-C_6)$-Alkyl, geradkettig oder verzweigt,

3) $(C_1-C_6)$-Alkyl, geradkettig oder verzweigt, worin die Kohlenstoffkette mit 1 oder 2 Sauerstoffatomen unterbrochen ist oder

4) $(C_3-C_6)$-Alkenyl, geradkettig oder verzweigt.

Die Erfindung betrifft weiterhin Verfahren zur Herstellung der neuen Xanthinderivate, wobei eine Ausführungsform beispielsweise darin besteht, daß man

a) Xanthinderivate der Formel I, wobei $R^2$ eine Alkylgruppe mit 1 bis 4 C-Atomen ist und $R^1$ und $R^3$ Wasserstoffatome bedeuten, mit einem Alkylierungsmittel der Formel IV

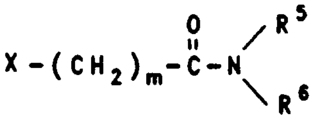 (IV)

oder einem Alkylierungsmittel der Formel $R^3$-X, wobei X Halogen wie Chlor, Brom oder Jod bedeutet oder eine Sulfonsäureester-Gruppierung ist, $R^3$, $R^4$, $R^5$, $R^6$, n und m die obengenannten Bedeutungen haben, wobei $R^3$ nicht Wasserstoffatom bedeutet, zu einen zweifach substituierten Xanthinderivat der Formel V umsetzt,

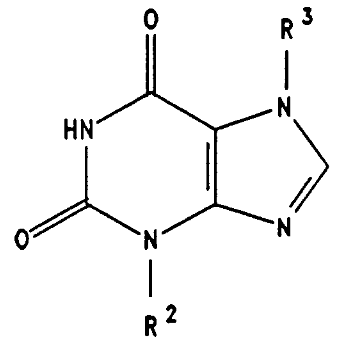

(V)

und gegebenenfalls das Xanthinderivat der Formel V mit einem der obengenannten Alkylierungsmittel oder einem Alkylierungsmittel der Formel $R^1$-X in Gegenwart eines basischen Mittels oder in Form ihrer Salze in der Position von $R^1$ alkyliert oder

b) 1,3-disubstituierte Xanthinderivate der Formel VI

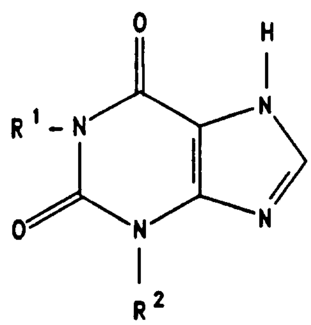

(VI)

wobei $R^1$ und $R^2$ die obengenannte Bedeutung haben, in Gegenwart basischer Mittel oder in Form ihrer Salze mit einem Alkylierungsmittel der Formel IV oder $R^3$-X umsetzt oder

c) Xanthinderivate der Formel I, in denen $R^1$ oder $R^3$ einen Oxoalkylrest bedeutet, mit üblichen Reduktionsmitteln zu den entsprechenden hydroxyalkylierten Xanthinderivaten reduziert.

Die Erfindung betrifft auch Arzneimittel, gekennzeichnet durch einen wirksamen Gehalt an mindestens einem Xanthinderivat der Formel I neben pharmazeutisch geeigneten und physiologisch verträglichen Trägerstoffen, Zusatzstoffen und/oder anderen Wirk- und Hilfsstoffen.

Die erfindungsgemäßen Arzneimittel können oral, topisch, rektal, intravenös oder gegebenenfalls auch parenteral appliziert werden. Die Applikation kann vor, nach und während der Unterbrechung der Blutzirkulation erfolgen.

Aufgrund der pharmakologischen Eigenschaften der erfindungsgemäßen Xanthinderivate können diese Verbindungen bei allen Operationen in der Klinik oder ambulanten Versorgung angewendet werden, in denen die Blutzirkulation an Geweben, Organen oder Extremitäten ganz oder teilweise unterbrochen wird. Ferner können diese Verbindungen auch bei Meniskusoperationen oder diagnostischen Eingriffen verwendet werden, wobei es zu einer völligen oder teilweisen Unterbrechung der Blutzirkulation im betroffenen Gewebe kommt. Insbesondere sind die erfindungsgemäßen Xanthine zur Prophylaxe und Verminderung von Nervenschädigungen, die nach arteriell oder venös bedingten Verschlüßen der Blutgefäße auftreten, geeignet.

Die Unterbrechung der Blutzirkulation sollte nicht viel länger als 240 Minuten dauern, bevorzugt von 5 bis 120 Minuten, insbesondere von 10 bis 30 Minuten. Die Zeit für die weitgehend komplikationslose Unterbrechung der Blutzirkulation hängt im wesentlichen davon ab, ob die Unterbrechung teilweise oder vollständig ist und welche Gewebe oder Organe von der Blutzirkulation abgeschnitten werden. Die zeitlichen Grenzen der Unterbrechung sind für den Fachmann einfach feststellbar.

Die Erfindung betrifft auch ein Verfahren zur Herstellung eines erfindungsgemäßen Arzneimittels, das dadurch gekennzeichnet ist, daß man mindestens ein Xanthinderivat der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z. B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Xanthinderivate der Formel I enthält. Bei festen Dosierungseinheiten, wie Tabletten, Kapseln, Dragees oder Suppositorien, kann diese Dosis bis zu etwa 300 mg, bevorzugt jedoch etwa 10 bis 100 mg betragen.

Für die Behandlung eines Patienten (70 kg), der sich einer gefäßchirurgischen Behandlung unterziehen muß, ist vor, während und nach der Operation eine Dosis von 400 bis 1200 mg pro Tag und Patient der erfindungsgemäßen Xanthinderivate der Formel I indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die erfindungsgemäßen Xanthinderivate der Formel I bei der Herstellung der vorgenannten galenischen Zubereitungsformen auch zusammen mit anderen geeigneten Wirkstoffen, beispielsweise Wirkstoffen, die freie Sauerstoffradikale abfangen, z. B. 1,5-Dihydro-4H-pyrazolo(3,4-d)-pyrimidin-4-on oder dem Enzym Superoxiddismutase, formuliert werden.

Beispiel 1

Herstellung von 1-(6-Oxoheptyl)-3-methyl-7-propylxanthin

2,1 g 3-Methyl-7-propylxanthin, 0,7 g 1-Chlor-heptanon-(6) und 1,4 g Kaliumcarbonat werden 7,5 Stunden in 15 ml Dimethylformamid bei 120 °C unter Rühren erhitzt. Nach Einengen bei vermindertem Druck gießt man den Rückstand in 100 ml $H_2O$, extrahiert dreimal mit je 100 ml $CHCl_3$, behandelt die vereinigten Chloroformextrakte nacheinander mit 1 n Natronlauge und Wasser und trocknet über Natriumsulfat. Der Einengungsrückstand wird in einer Säule mit Kieselgel 60 und Chloroform/Ethanol (9 : 1) als Laufmittel chromatographiert. Nach Rekristallisation aus Petrolether/Diisopropylether (3 : 1) erhält man 2,45 g (= 76,6 % Ausbeute) 1-(6-Oxoheptyl)-3-methyl-7-propylxanthin vom Schmelzpunkt 69 - 70 °C.
$C_{16}H_{24}N_4O_3$ = 320,40

| | | | |
|---|---|---|---|
| ber.: | C 59,98 % | H 7,55 % | N 17,49 % |
| gef.: | C 59,81 % | H 7,53 % | N 17,65 % |

Analog zu Beispiel 1 werden die nachfolgenden Verbindungen hergestellt.

Beispiel 2

Aus 3-Ethyl-7-propylxanthin und 1-Chlor-hexanon-(5) wird 3-Ethyl-1-(5-oxohexyl)-7-propylxanthin (Schmelzpunkt 81 - 82 °C) hergestellt.

Beispiel 3

Aus 3-Methyl-7-propylxanthin und 1-Chlor-pentanon-(4)-ethylen Ketal wird zunächst das Ethylen Ketal von 3-Methyl-1-(4-oxopentyl)-7-propylxanthin erhalten. Dieses wird durch Hydrolyse (1 h/70 °C) in Methanol/Wasser (Volumenverhältnis 8:2) in Gegenwart von 33%iger Schwefelsäure in 3-Methyl-1-(4-oxopentyl)-7-propylxanthin (Schmelzpunt 67 - 68 °C) übergeführt.

Beispiel 4

Aus 1,3-Diethyl-xanthin und 1-Chlor-hexanon-(5) wird 1,3-Diethyl-7-(5-Oxohexyl)-xanthin (Schmelzpunkt 68 - 69 °C) hergestellt.

Beispiel 5

1-(4-Oxo-3-methylpentyl)-3-methyl-7-propyl-xanthin

a) 1-Chlor-3-methylpentan-4-on
Zu einer Lösung von
128,1 g (1 mol) α-Acetyl-γ-butyrolacton in 500 ml absol. Ethanol gibt man unter Rühren portionsweise
23,0 g (0,1 mol) Natrium. Nach erfolgter vollständiger Auflösung des Natriums wird tropfenweise
126,1 g (1 mol) Dimethylsulfat zugegeben, wobei sich die Reaktionsmischung innerhalb von 30 Minuten auf Rückflußtemperatur erwärmt. Nach 1-stündigem Erhitzen bei Rückflußtemperatur und Einengen im Vakuum wird der ölige Rückstand über eine Kolonne fraktioniert destilliert. Es fallen 106 g α-Acetyl-α-methyl-γ-butyrolacton an ($Kp_{14}$ = 119 - 122 °C), das direkt einer sauren Hydrolyse unterworfen wird. Dazu wird das obige Lacton in eine siedende Mischung von
200 ml konz. Salzsäure und
250 ml Wasser zugetropft und das Umsetzungsprodukt unter $CO_2$-Entwicklung in eine gekühlte Vorlage überdestilliert. Extraktion mit Ether, Trocknen über Natriumsulfat und Destillation des organischen Rückstandes über eine Kolonne ergibt 92 g 1-Chlor-3-methylpentan-4-on mit dem $Kp_{16}$ = 69 - 70 °C.
b) 1-(4-Oxo-3-methylpentyl)-3-methyl-7-propyl-xanthin
25,0 g (0,12 mol) 3-Methyl-7-propylxanthin werden zusammen mit
16,2 g (0,12 mol) 1-Chlor-3-methylpentan-4-on (Beispiel 5a) und
16,6 g (0,12 mol) Kaliumcarbonat in 300 ml Dimethylformamid 8 Stunden bei 140 °C gerührt. Die Lösung wird anschließend unter vermindertem Druck eingedampft, mit verdünnter Natronlauge alkalisiert und mehrmals mit Chloroform extrahiert. Die vereinigten Chloroformextrakte wäscht man mit wenig Wasser neutral, trocknet über Natriumsulfat und destilliert das Lösemittel im Vakuum ab, wobei festes Rohprodukt anfällt, das aus Diisopropylether umkristallisiert wird.
Ausbeute: 25,9 g (70 % d. Th.)
Schmelzpunkt: 63 - 64 °C
$C_{15}H_{22}N_4O_3$ (MG = 306,4)

| Analyse: | Berechnet: | C 58,81 % | H 7,24 % | N 18,29 %; |
|---|---|---|---|---|
| | Gefunden: | C 59,02 % | H 7,28 % | N 18,22 %. |

Beispiel 6

1-(4,4-Ethylendioxy-3-methylpentyl)-3-methyl-7-propyl-xanthin

a) Darstellung des 1,3-Dioxolans von 1-Chlor-3-methyl-pentanon-4
Dazu werden
40,4 g (0,3 mol) 1-Chlor-3-methylpentan-4-on,
18,6 g (0,3 mol) Ethylenglycol sowie
0,5 g p-Toluolsulfonsäure in
300 ml Toluol gelöst und im Wasserabscheider am Rückfluß erhitzt. Die abgeschiedene organische Phase wird mit verd. Natronlauge versetzt und mit Ether ausgeschüttelt. Trocknen der organischen Phase über Natriumsulfat und fraktionierte Destillation des öligen Rückstandes ergibt 37,6 g des Dioxolans (70 % der Th.) mit einem $Kp_{26}$ = 47 - 50 °C.
b) 1-(4,4-Ethylendioxy-3-methylpentyl)-3-methyl-7-propylxanthin
Analog Beispiel 5 werden
12,5 g (0,06 mol) 3-Methyl-7-propylxanthin mit
12,0 g (0,067 mol) des Dioxolans (Beispiel 6a) in Gegenwart von
9,3 g (0,067 mol) Kaliumcarbonat in
150 ml Dimethylformamid umgesetzt.

Ausbeute: 18,2 g (86 % d. Th.).
$C_{18}H_{28}N_4O_4$ (MG = 350,4)
Schmelzpunkt: 35 - 37 °C

| | | | | |
|---|---|---|---|---|
| Analyse: | Berechnet:<br>Gefunden: | C 58,27 %<br>C 58,35 % | H 7,48 %<br>H 7,70 % | N 15,99 %;<br>N 15,82 %. |

Analog zu Beispiel 5 werden die nachfolgenden Verbindungen hergestellt.

Beispiel 7

Aus 3-Methyl-7-allylxanthin und 1-Chlor-3-methylpentanon-4 wird 1-(4-Oxo-3-methylpentyl)-3-methyl-7-allyl-xanthin (Schmelzpunkt 73 - 74 °C) hergestellt.

Beispiel 8

Aus 3-Ethyl-7-(2-methoxyethyl)-xanthin und 1-Chlor-3-methylpentanon-4 wird 1-(4-Oxo-3-methylpentyl)-3-ethyl-7-(2-methoxyethyl)-xanthin (Schmelzpunkt 83 - 84 °C) hergestellt.

Beispiel 9

1-(4-Hydroxy-3-methylpentyl)-3-methyl-7-propyl-xanthin

Zu einer Suspension von
9 g (0,029 mol) 1-(4-Oxo-3-methylpentyl)-3-methyl-7-propyl-xanthin (Beispiel 5b) in 100 ml Methanol werden unter Rühren
1,1 g (0,028 mol) Natriumborhydrid zugesetzt. Nach 2-stündigem Rühren wird unter vermindertem Druck eingedampft, der Rückstand in Chloroform aufgenommen, nacheinander mit verdünnter Natronlauge und Wasser gewaschen, über Natriumsulfat getrocknet und zur Trockne eingeengt. Die Ausbeute an dem hochviskosen Endprodukt beträgt 7,5 g (84 % d. Th.).
$C_{15}H_{24}N_4O_3$ (MG = 308,4)

| | | | | |
|---|---|---|---|---|
| Analyse: | Berechnet:<br>Gefunden: | C 58,42 %<br>C 58,50 % | H 7,84 %<br>H 7,86 % | N 18,17 %;<br>N 17,97 %. |

Beispiel 10

1-Ethoxymethyl-3-methyl-7-dimethylamino-carbonylmethylxanthin

a) 3-Methyl-7-dimethylaminocarbonylmethylxanthin
Zu einer Lösung von
2 g (0,05 mol) Natriumhydroxyd in
40 ml Wasser gibt man bei 70 °C
8,3 g (0,05 mol) 3-Methyl-xanthin und anschließend
6,45 g (0,053 mol) 2-Chlor-dimethylacetamid und rührt weitere 7 Stunden bei dieser Temperatur. Nach dem Abkühlen wird der ausgefallene Rückstand abgesaugt, mit Wasser und Methanol gewaschen und in 2 n-NaOH gelöst. Beim Einstellen des pH-Wertes mit 2 n-$H_2SO_4$ auf 10 fällt ein Niederschlag aus, der nach 2 stündigem Rühren abgesaugt, mit Wasser gewaschen und bei 100 °C getrocknet wird.
Ausbeute: 8,0 g (64 % d. Th.).
Schmelzpunkt: 336 °C.
$C_{10}H_{13}N_4O_3$ (MG = 251,2).

| Analyse: | Berechnet:<br>Gefunden: | C 47,81 %<br>C 47,92 % | H 5,22 %<br>H 5,10 % | N 27,88 %;<br>N 27,36 %. |
|---|---|---|---|---|

2b) 1-Ethoxymethyl-3-methyl-7-dimethylamino-carbonylmethyl-xanthin
Zur Darstellung des Natriumsalzes von Beispiel 10a wird
0,92 g (0,04 mol) Natrium in
160 ml absol. Methanol gelöst und mit
10 g (0,04 mol) Verbindung aus Beispiel 10a versetzt. Nach 15 min Erhitzen auf Rückflußtemperatur werden nach Zugabe von
5,3 g (0,056 mol) Ethoxymethylchlorid in
150 ml Acetonitril nochmals 8 Stunden weiter erhitzt. Abkühlen und Einengen der Lösung ergibt einen festen Rückstand, der in Methylenchlorid gelöst und dann mit verd. NaOH versetzt und anschließend mit Wasser gewaschen wird. Einengen der über Natriumsulfat getrockneten Phase und Umkristallisation aus Ethanol führt zu 1-Ethoxymethyl-3-methyl-7-dimethylamino-carbonylmethyl-xanthin.
Ausbeute: 7,1 g (57 % d. Th.).
Schmelzpunkt: 183 - 184 °C.
$C_{13}H_{19}N_5O_4$ (MG = 309,3).

| Analyse: | Berechnet:<br>Gefunden: | C 58,48 %<br>C 58,19 % | H 6,19 %<br>H 6,25 % | N 22,64 %;<br>N 22,68 %. |
|---|---|---|---|---|

Beispiel 11

1,7-Bis-(piperazinocarbonylmethyl)-3-methyl-xanthin

a) Benzyl-1-piperazin-carboxylat
194 g (1 M) Piperazinhexahydrat werden unter Rühren mit 250 ml Wasser und 500 ml Methanol versetzt und mit etwa 163 ml konzentrierter Salzsäure auf pH 3 eingestellt. Dann wird auf 25 °C abgekühlt und unter Rühren werden 100 g (0,59 M) Chlorameisensäurebenzylester zugetropft und dabei wird der pH-Wert zwischen 3 und 4,5 mit Natronlauge (4 M; etwa 230 ml) gehalten. Anschließend werden am Rotationsverdampfer ca. 700 ml abgezogen, der Rückstand mit Wasser gewaschen, mit etwa 1,5 ml konzentrierter HCl sauer gestellt und zweimal mit je 200 ml Toluol extrahiert.
Die wäßrige Phase wird abgekühlt und mit ca. 115 ml 12,5 N NaOH auf pH 12 gestellt. Das sich abscheidende Öl mehrmals mit Toluol extrahiert und der Extrakt über $K_2CO_3$ getrocknet und unter vermindertem Druck eingeengt.
Ausbeute: 74 g

| Analyse | Berechnet:<br>Gefunden: | C 65,43 %<br>C 65,32 % | H 7,32 %;<br>H 7,65 %. |
|---|---|---|---|

$C_{12}H_{16}N_2O_2$ (MG = 220,2).
b) 4-Chloracetyl-1-benzyloxycarbonyl-piperazin
110 g (0,5 M) Benzyl-1-piperazincarboxylat werden unter Rühren hergestellt, wie bei a), werden bei etwa -60 °C mit 150 ml Methylenchlorid versetzt und unter Rühren 18,3 ml (0,23 M) Chloracetylchlorid in 50 ml Methylenchlorid innerhalb von 90 min zugetropft. Anschließend wird langsam auf 25 °C erwärmt und 2 Stunden weitergerührt. Die Lösung wird nacheinander mit verdünnter Salzsäure, verdünnter Natronlauge und Wasser gewaschen, über $Na_2SO_4$ getrocknet und zur Trockene eingeengt. Die Ausbeute an dem hochviskosen Endprodukt beträgt 63 g.

| Analyse | Berechnet:<br>Gefunden: | C 56,66 %<br>C 56,53 % | H 5,77 %<br>H 5,8 % | Cl 11,95 %<br>Cl 11,47 % | N 9,44 %;<br>N 9,45 %. |
|---|---|---|---|---|---|

$C_{14}H_{17}N_2O_3Cl$ (MG = 296,7).

c) 3-Methyl-1,7-bis-(4-benzyloxycarbonyl-1-piperazinylcarboxy-methyl)-xanthin

5,4 g (0,0326 M) 3-Methylxanthin werden zusammen mit 21 g (0,0708 M) der Verbindung erhalten nach b) in Gegenwart von 10,7 g (0,0775 M) $K_2CO_3$ in 100 ml Dimethylformamid (DMF) 7 Stunden bei 120 - 130 °C gerührt, dann filtriert und mit DMF gewaschen. Das unter verminderten Druck eingeengte Filtrat ergibt 25 g eines hochviskosen Rohprodukts.

d) 1,7-Bis-(piperazinocarbonylmethyl)-3-methyl-xanthin

20 g des in c) erhaltenen Rohprodukts werden in Gegenwart von 2 g Palladium/Kohle in 200 ml Eisessig unter Rühren bei etwa 75 °C 8 Stunden lang mit $H_2$ begast. Das Filtrat wird eingeengt und nach dem Trocknen im Exsikkator in 150 ml Ethanol bei 70 °C gelöst. Nach Behandeln mit Aktivkohle und Versetzen mit ethanolischer HCl fällt das Produkt in Form des Dihydrochlorids aus.

Ausbeute: 11 g (80 % d. Th.)

Schmelzpunkt: 338 °C

$C_{18}H_{26}N_8O_4$ (MG = 418,46).

Die nachfolgende Tabelle 1 gibt einen Überblick über die hergestellten Verbindungen.

Tabelle I: Verbindungen gemäß Formel I

| Beispiel | $R^1$ | $R^2$ | $R^3$ | Schmelzpunkt °C |
|---|---|---|---|---|
| 1 | 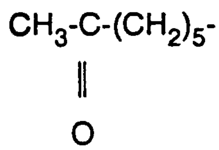 | $CH_3$- | $C_3H_7$- | 69 - 70 |
| 2 | 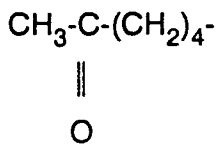 | $C_2H_5$- | $C_3H_7$- | 81 - 82 |
| 3 | 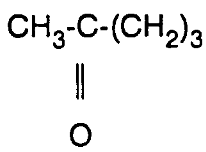 | $CH_3$- | $C_3H_7$- | 67 - 68 |
| 4 | $C_2H_5$- | $C_2H_5$- | 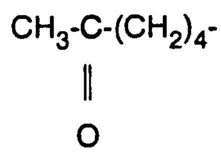 | 68 - 69 |
| 5 | 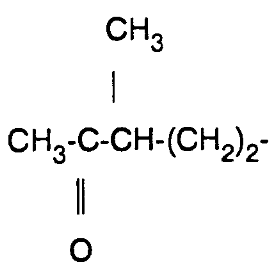 | $CH_3$- | $C_3H_7$- | 63 - 64 |
| 6 | 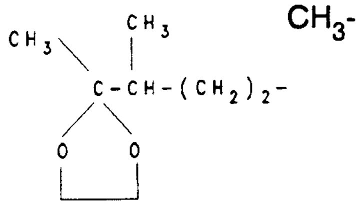 | $CH_3$- | $C_3H_7$- | 35 - 37 |

| Beispiel | $R^1$ | $R^2$ | $R^3$ | Schmelzpunkt °C |
|---|---|---|---|---|
| 7 | 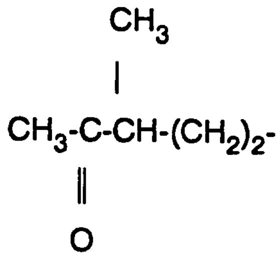 | $CH_3$- | $CH_2$=CH-$CH_2$- | 73 - 74 |
| 8 | 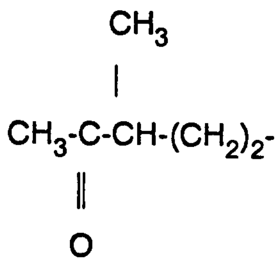 | $C_2H_5$- | $CH_3$-O-$(CH_2)_2$- | 83 - 84 |
| 9 | 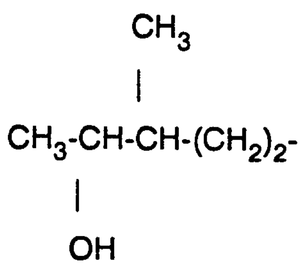 | $CH_3$- | $C_3H_7$- | Öl |
| 10 | $CH_3$-$CH_2$-O-$CH_2$- | $CH_3$- | 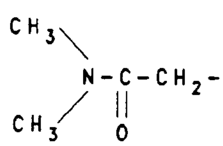 | 183-184 |
| 11 | 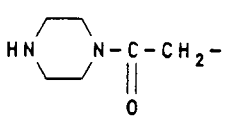 | $CH_3$- | 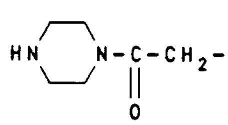 | 338 (2HCl) |

Beispiel 12

Pharmakologische Prüfungen

Wirkung auf den Sauerstoffpartialdruck im Gewebe des Gehirns

In dem hier angewendeten Modell wird die Fähigkeit eines Präparates gemessen, den Sauerstoffpartialdruck im Hirncortex unter pysiologischen und/oder pathologischen Bedingungen zu steigern, was als Zeichen eines verbesserten Stoffwechsels im Gehirn interpretiert werden kann.

Versuchstier: Ratte

| | |
|---|---|
| Stamm: | genetische Hochdruckratte (Mollegaard/Stroke Prone, Dänemark) |
| Gewicht: | 280 - 300 g |
| Geschlecht: | männlich |
| Applikation: | Dauerinfusion 10 min. |
| | 0.1 ml/min. |
| | 0.1 - 1 mg/kg |

Durchführung:

Das Versuchstier wird mit 60 mg/kg i. p. Na-Pentobarbital (Sigma) narkotisiert.

Präparation:

Zur Messung der Blutgase und des Blutdrucks wird die Arteria femoralis und für die Infusion der Testsubstanz die Vena femoralis katheterisiert.

Danach wird die Trachea intubiert und der Kopf des Tieres in einem stereotaktischen Rahmen fixiert. Der Schädelknochen wird freipräpariert und eine kreisförmige Öffnung von ca. 3 mm trepaniert; dabei bleibt die Dura mater intakt.

Die Mehrdraht-Oberflächenelektrode wird vor Versuchsbeginn mit 2 % bzw. 12 % Sauerstoff angereichert (Eichgerät für gaspartielle Messungen $O_2$ und $CO_2$, Fa. Rhema).
Mittels Mikromanipulator (Fa. Brinkmann, Mannheim) wird die Elektrode leicht auf die trepanierte Hirnoberfläche aufgesetzt und mit 0,9 % NaCl umgeben. Nach Erreichen einer stabilen physiologischen Ausgangslage wird der Zeitverlauf der $O_2$ und $CO_2$-Werte aufgezeichnet.
Für die Dauer von 10 min. wird die Prüfsubstanz (0.1 ml/min.) in der Normaldosierung von 1 mg/kg/min infundiert. Die Anzahl der Versuchstiere für eine Prüfsubstanz sind jeweils 5 bis 6.

Tabelle 2 zeigt die Ergebnisse in Prozent im Vergleich zu einer Kontrolle, bei der statt Prüfsubstanz physiologische Kochsalzlösung infundiert wird.

Tabelle 2

| Verbindung gemäß Beispiel Kontrolle | Veränderung des Sauerstoffpartialdruckes im Vergleich zur Kontrolle (%) 0 |
|---|---|
| 1 | + 34 |
| 2 | + 23 |
| 3 | + 13 |
| 4 | + 14 |
| 5 | + 40 |
| 6 | + 33 |
| 7 | + 15 |
| 8 | + 15 |
| 9 | + 9 |
| 10 | + 10 |
| 11 | + 12 |

**Patentansprüche**

**1.** Verwendung von mindestens einem Xanthinderivat der Formel I

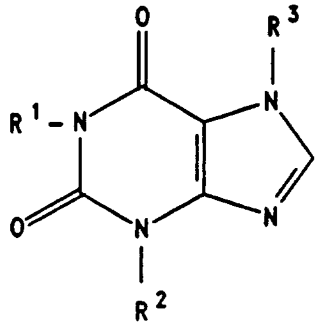

(I)

wobei $R^2$ eine Alkylgruppe mit 1 bis 4 C-Atomen ist und

a) mindestens einer der Reste $R^1$ und $R^3$ einen geradkettigen oder verzweigten Rest der Formel II

$R^4$

|

$-(CH_2)_n-CH-A-CH_3$ (II)

darstellt, worin A

1) -CH- ,

|

OH

2) -C- oder

||

O

3) 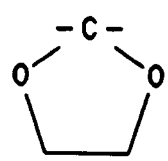

bedeutet,

$R^4$

1) Wasserstoffatom oder

2) $(C_1-C_4)$-Alkylgruppe ist und

n eine ganze Zahl von 0 bis 5 darstellt oder

b) mindestens einer der Reste $R^1$ und $R^3$ ein Rest der Formel III

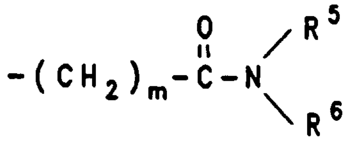 (III)

darstellt, worin
$R^5$ und $R^6$ gleich oder verschieden sind und unabhängig voneinander ein Wasserstoffatom oder eine $(C_1-C_4)$-Alkylgruppe sind, oder $R^5$ und $R^6$ bilden zusammen mit dem Stickstoffatom an das sie gebunden sind, einen 5 bis 7-gliedrigen Ring, der gegebenenfalls anstelle eines der ringständigen C-Atome ein weiteres Heteroatom in Form von Sauerstoff oder Stickstoff enthält, wobei das weitere Stickstoffatom auch durch eine $(C_1-C_4)$-Alkylgruppe substituiert sein kann,
m die ganze Zahl 1 oder 2 ist
und der gegebenenfalls vorhandene andere Rest $R^1$ oder $R^3$ steht für
1) Wasserstoffatom,
2) $(C_1-C_6)$-Alkyl, geradkettig oder verzweigt,
3) $(C_1-C_6)$-Alkyl, geradkettig oder verzweigt, worin der Kohlenstoff mit 1 oder 2 nicht aneinander gebundenen Sauerstoffatomen unterbrochen ist oder
4) $(C_3-C_6)$-Alkenyl, geradkettig oder verzweigt,
wobei 1-(5-Oxohexyl)-3-methyl-7-n-propylxanthin ausgenommen ist, zur Herstellung von Arzneimitteln zur Prophylaxe und Behandlung von Nervenschädigungen nach Unterbrechung der Blutzirkulation.

**2.** Verwendung gemäß Anspruch 1, dadurch gekennzeichnet, daß man mindestens ein Xanthinderivat der Formel I, wobei
$R^2$ eine Alkylgruppe mit 1 bis 2 C-Atomen ist und
mindestens einer der Reste $R^1$ und $R^3$ einen geradkettigen oder verzweigten Rest der Formel II darstellt, worin A

1) 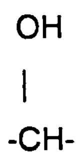 ,

2) 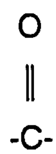 oder

3) 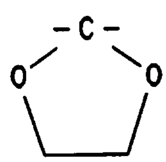

bedeutet,
$R^4$ Wasserstoffatom oder eine $(C_1-C_2)$-Alkylgruppe ist und
n eine ganze Zahl von 2 bis 5 darstellt
und der gegebenenfalls vorhandene andere Rest $R^1$ oder $R^3$ steht für
1) Wasserstoffatom,
2) $(C_1-C_6)$-Alkyl, geradkettig oder verzweigt,

3) ($C_1$-$C_6$)-Alkyl, geradkettig oder verzweigt, worin die Kohlenstoffkette mit 1 oder 2 Sauerstoffatomen unterbrochen ist,
4) ($C_3$-$C_6$)-Alkenyl, geradkettig oder verzweigt, einsetzt,
wobei 1-(5-Oxohexyl)-3-methyl-7-n-propylxanthin ausgenommen ist.

**3.** Verwendung gemäß Anspruch 1 oder 2 zur Verlängerung der Operationszeit nach der Unterbrechung der Blutzirkulation.

**4.** Verwendung gemäß einem oder mehreren der Ansprüche 1 bis 3 zur Prophylaxe und Verminderung von Nervenschädigungen, die nach arteriell oder venös bedingter Verschlüße der Blutgefäße auftreten.

**5.** Verwendung gemäß einem oder mehreren der Ansprüche 1 bis 4 zur Erhöhung des Sauerstoffpartialdrucks im Gehirn.

**6.** Verwendung gemäß einem oder mehreren der Ansprüche 1 bis 5 zur Applikation vor, nach oder während einer Operation, bei der es zu einer Unterbrechung der Blutzirkulation kommt.

**7.** Xanthinderivat der Formel I

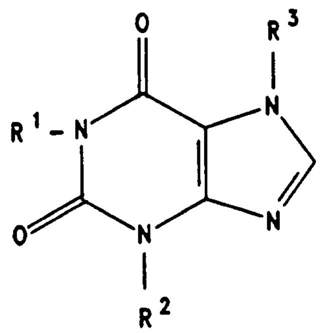

( I )

wobei $R^2$ eine Alkylgruppe mit 1 bis 4 C-Atomen ist und
a) mindestens einer der Reste $R^1$ und $R^3$ einen geradkettigen oder verzweigten Rest der Formel II

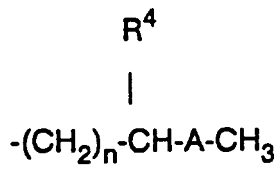

(II)

darstellt, worin A

1) 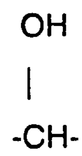 ,

2) 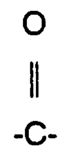 oder

3) 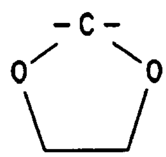

bedeutet,

$R^4$ eine $(C_1\text{-}C_4)$-Alkylgruppe ist und

n eine ganze Zahl von 2 bis 5 darstellt oder

b) mindestens einer der Reste $R^1$ und $R^3$ einen Rest der Formel III

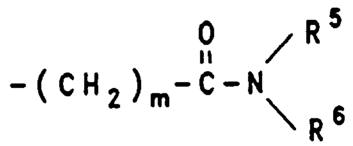 (III)

darstellt, worin

$R^5$ und $R^6$ gleich oder verschieden sind und unabhängig voneinander ein Wasserstoffatom oder eine $(C_1\text{-}C_4)$-Alkylgruppe sind oder $R^5$ und $R^6$ bilden zusammen mit dem Stickstoffatom an das sie gebunden sind, einen 5 bis 7-gliedrigen Ring, der gegebenenfalls anstelle eines der ringständigen C-Atome ein weiteres Heteroatom in Form von Sauerstoff oder Sticktoff enthält, wobei das weitere Stickstoffatom auch durch eine $(C_1\text{-}C_4)$-Alkylgruppe substituiert sein kann,

m die ganze Zahl 1 oder 2 ist

und der gegebenenfalls vorhandene Rest $R^1$ oder $R^3$ steht für

1) Wasserstoffatom,

2) $(C_1\text{-}C_6)$-Alkyl, geradkettig oder verzweigt,

3) $(C_1\text{-}C_6)$-Alkyl, geradkettig oder verzweigt, worin der Kohlenstoff mit 1 oder 2 Sauerstoffatomen unterbrochen ist oder

4) $(C_3\text{-}C_6)$-Alkenyl, geradkettig oder verzweigt.

**8.** Verfahren zur Herstellung der Xanthinderivate der Formel I gemäß Anspruch 7, dadurch gekennzeichnet, daß man

a) Xanthinderivate der Formel I, wobei $R^2$ eine Alkylgruppe mit 1 bis 4 C-Atomen ist und $R^1$ und $R^3$ Wasserstoffatom bedeutet, mit einem Alkylierungsmittel der Formel IV

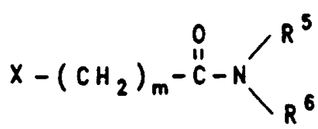 (IV)

oder einem Alkylierungsmittel der Formel $R^3$-X, wobei X Halogen wie Chlor, Brom oder Jod bedeutet oder eine Sulfonsäureester-Gruppierung ist, $R^3$, $R^4$, $R^5$, $R^6$, n und m die in Anspruch 1 genannte Bedeutung haben, wobei $R^3$ nicht Wasserstoffatom bedeutet, zu einen zweifach substituierten Xanthinderivat der Formel V umsetzt,

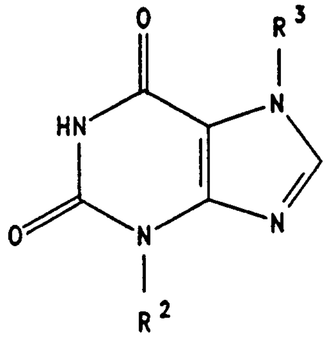

(V)

und gegebenenfalls das Xanthinderivat der Formel V mit einem der obengenannten Alkylierungsmittel oder einem Alkylierungsmittel der Formel $R^1$-X in Gegenwart eines basischen Mittels oder in Form ihrer Salze in der Position von $R^1$ alkyliert oder
b) 1,3-disubstituierte Xanthinderivate der Formel VI

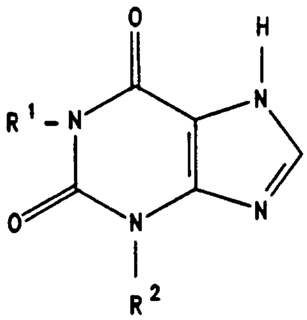

(VI)

wobei $R^1$ und $R^2$ die in Anspruch 1 genannte Bedeutung haben, in Gegenwart basischer Mittel oder in Form ihrer Salze mit einem Alkylierungsmittel der Formel IV oder $R^3$-X umsetzt oder
c) Xanthinderivate der Formel I, in denen $R^1$ oder $R^3$ einen Oxoalkylrest bedeutet, mit üblichen Reduktionsmitteln zu den entsprechenden hydroxyalkylierten Xanthinderivaten reduziert.

**9.** Arzneimittel, gekennzeichnet durch einen wirksamen Gehalt von mindestens einem Xanthinderivat der Formel I gemäß Anspruch 7 oder erhalten gemäß Anspruch 8, neben pharmazeutisch geeigneten und physiologisch verträglichen Trägerstoffen, Verdünnungsmitteln und/oder anderen Wirk- oder Hilfsstoffen.

**10.** Verfahren zur Herstellung eines Arzneimittels gemäß Anspruch 9, dadurch gekennzeichnet, daß man mindestens ein Xanthinderivat der Formel I gemäß Anspruch 7 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.